# EUROPEAN PATENT APPLICATION

(11) **EP 1 632 195 A1**
(43) Date of publication of application: **08.03.2006**
(21) Application number: 04022600.3
(22) Date of filing: 22.09.2004
(51) Int. Cl.: A61B 19/04

(54) **Strippable multi-layer medical glove**

(30) Priority: 07.09.2004 US 934535
(71) Applicant: Motex Healthcare Corp., Taipei (TW)
(72) Inventor: Cheng, Yung-Chu, Tapei, Taiwan (TW)
(74) Representative: Hartz, Nikolai

(57) **Abstract**

It is a medical glove being a plurality of layers (individual gloves) sleeved one over another in such a way that the outer layers can be stripped to unveil new clean exteriors.

## Description

### BACKGROUND AND SUMAMRY OF THE INVENTION

The present invention relates to a medical glove and more particularly, to a strippable multi-layer medical glove that is strippable layer by layer for repeated use.

A regular medical glove is formed of latex or plastic material. In order to prevent contamination, a medical glove has to be thrown away after each use. However, when there are a lot of patients, it is inconvenient for a doctor or a medical person to be replacing gloves before every examination. Doing so wastes a lot of time and may lead to serious problems in case of an emergency.

The present invention has been accomplished under the above circumstances in view, thus it is the main objective of the present invention to provide a medical glove for repeated use. To accomplish such objective, the present invention is comprised of a plurality of individual gloves, hereon referred to as layers, sleeved one over another in such a way that outer glove can be removed to unveil a clean new surface. With such a glove, the user can use the same glove on multiple patients without infecting one patient with whatever maybe present in another. Not only does it save the user's time and money, it does so while being as clean as a new glove. Furthermore, it gives the user option of removing different numbers of layers to suit the needs in different situations.

### BRIEF DESCRIPTION OF THE DRAWING

Figure 1 is a sectional view of the outer layer of the strippable multi-layer medical glove, as according to the present invention.
Figure 2 is a sectional view showing the first intermediate layer formed on the outer layer, as according to the present invention.
Figure 3 is a sectional view showing the second intermediate layer formed on the first intermediate layer over the outer layer, as according to the present invention.
Figure 4 is a sectional view showing the third intermediate layer formed on the second intermediate layer outside the outer layer and the first intermediate layer, as according to the present invention.
Figure 5 is a sectional view showing an inner layer formed on the third intermediate layer outside the outer layer, the first intermediate layer and the second intermediate layer, as according to the present invention.

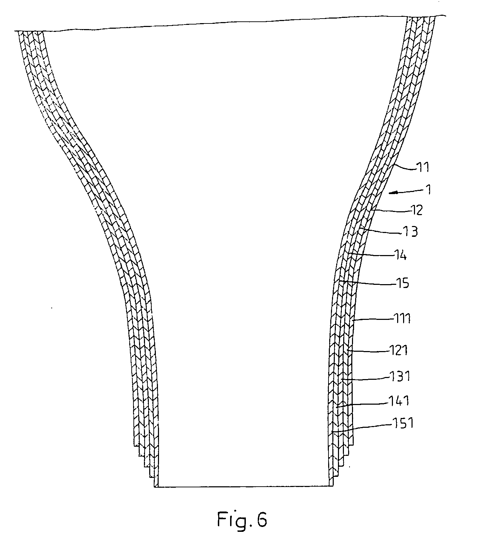

Figure 6 corresponds to Figure 5, showing the individual gloves turned inside out.

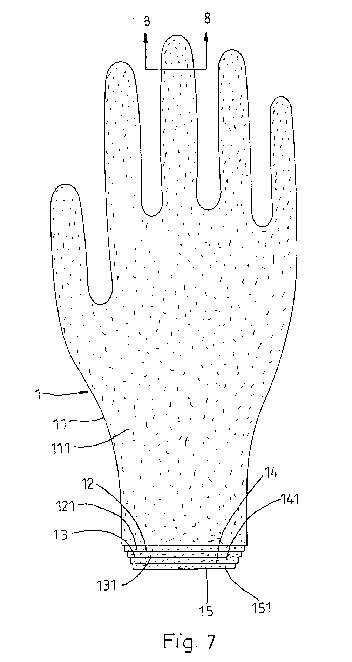

Figure 7 is a plain view of the strippable multi-layer medical glove as according to the present invention.
Figure 8 is a sectional view at an enlarged scale taken along line 8-8 of Figure 7.

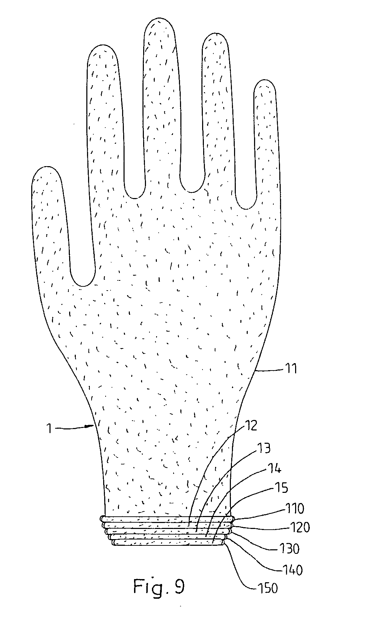

Figure 9 is a plain view of an alternative form of the strippable multi-layer medical glove, which shows a bead formed in the cuff of each layer, as according to the present invention.

### DETAILED DESCRIPTION OF THE INVENTION

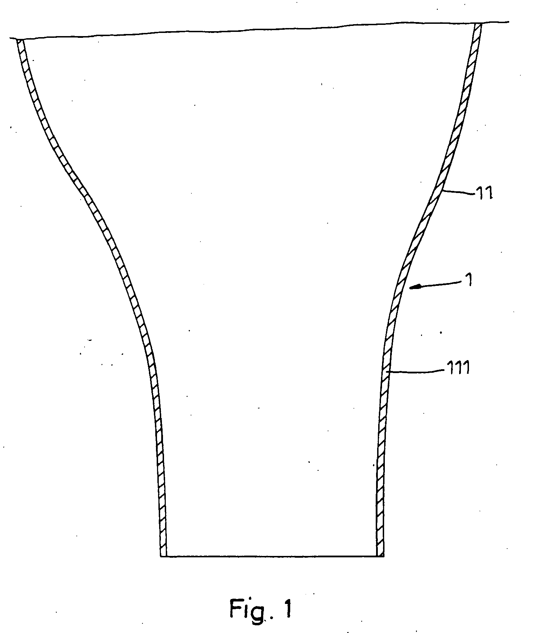

Referring to Figure 1, a strippable multi-layer medical glove **1** is shown comprising of a plurality of layers **11**~**15** sleeved one over another. The layers **11**~**15** include an outer layer **11**, an inner layer **15**, and a plurality of intermediate layers **12**~**14** sleeved one over another between the outer layer **11** and the inner layer **15**. The length of the cuff **151** of the inner layer **15** is the longest. The length of the cuff **111** of the outer layer **11** is the shortest. The cuffs **121,131,141** of the intermediate layers **12**~**14** are respectively from the shortest to the longest, but are shorter than the length of the cuff **151** of the inner glove **15** and longer than the length of the cuff **111** of the outer glove **11**.

After one use, the outermost layer **11** of the strippable multi-layer medical glove **1** is then stripped from the glove (apart from layers **12**~**15**), to unveil the clean new surface of layer **12**. It is then that the strippable multi-layer medical glove **1** can be used as a new glove.

The inner layer **15** has a thickness of about 0.08mm (fits ASTM D3578-00). The other layers **11**~**14** have a thickness of within 0.03mm~0.1mm. For example, if the thickness of the inner layers 15 is 0.08mm and the thickness of the other layers **11**~**14** are 0.05mm, then the total thickness of the strippable multi-layer medical glove **1** is 0.28mm. A glove having the thickness of 0.2mm is comfortable and convenient.

To make this glove, a glove mold is first immersed into a bath to be covered with a coat of latex or plastic substance. The mold is then dried to turn the coat into the outer layer **11** (see Figure 1).

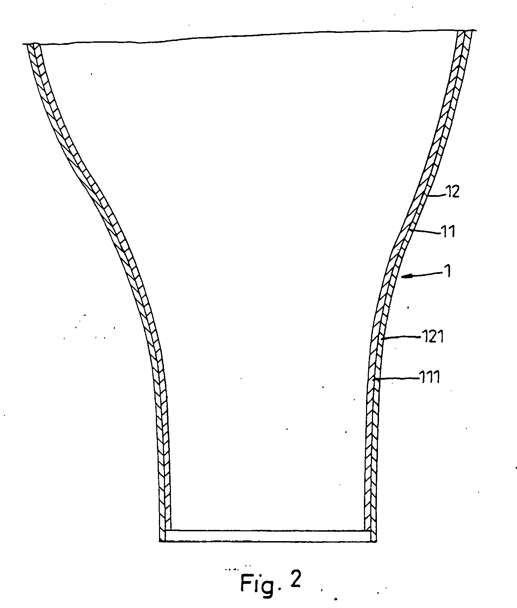

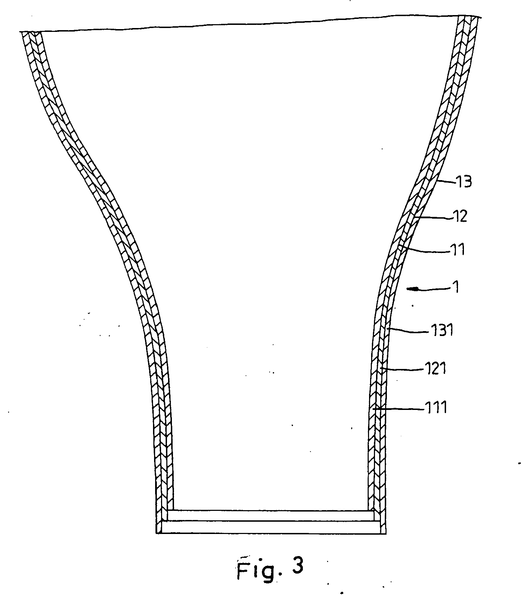

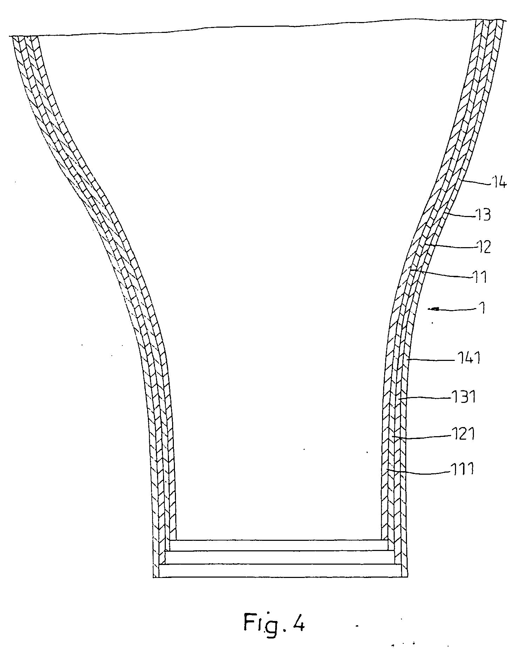

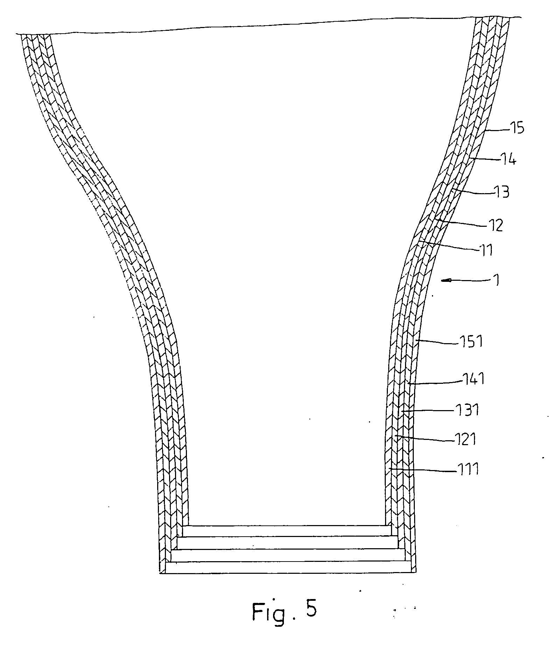

After the outer layer's formation, the mold is then covered with a coat of stripping substance over the outer layer, and is immersed again into the bath. The second coat then dries to become the first intermediate layer **12** (see Figure 2). This process of coating with stripping substance and coating with latex or plastic substance is then repeated to form successively the following intermediate layers **13**~**15** (see Figures 3~5). After all layers are formed, the glove is almost made.

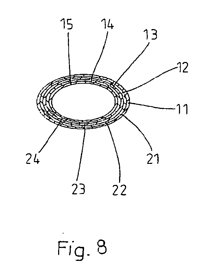

Next, the glove is removed from the mold and turned inside out. Thin layers of stripping material **21**~**24** are respectively formed between each two adjacent individual gloves (see Figure 8). At this point, two processes can be done (not exclusive of one another). One is that the inner layer can then be treated to be anti-microbial. Another is that after the formation of the layers and before vulcanizing the cuff **111**~**151** of each of the individual layers **11**~**15**, the cuffs **111**~**151** may be rolled up to form respective beads **110**~**150**. The glove is then finished.

While only one embodiment of the present invention has been shown and described, it will be understood that various modifications and changes could be made thereunto without departing from the spirit and scope of the invention disclosed. For example, the individual gloves may be so arranged that the outer glove has the longest cuff and the inner glove has the shortest cuff.

As indicated above, the invention provides a strippable multi-layer medical glove that achieves the following advantages:
1. Due to the fact that the user can remove one individual layer after each use of the strippable multi-layer medical glove, the strippable multi-layer medical glove can be used repeatedly. Therefore, the invention saves much time and cost from replacing the glove.
2. Adaptable for use in different situations, the glove can be stripped of multiple layers by the user for meet the user's needs.
3. Unless left with the last layer, the inner layer, at least two layers of protection, equivalent with the protection of two gloves, are protecting the hand. This is providing safer protection.

## Claims

1. A strippable multi-layer medical glove comprising of a plurality of individual layers sleeved one over another, in such a way being the topmost longer-cuffed outer layer sandwiching the intermediate layers with the bottommost inner layer, that the user can remove one layer after one use and go on with the next use.

2. The strippable multi-layer medical glove as claimed in claim 1, wherein said inner layer has a thickness of about 0.08mm; said intermediate gloves and said outer glove each have a thickness within 0.03mm~0.10mm.
